# EUROPEAN PATENT APPLICATION

(11) **EP 3 050 554 A1**
(43) Date of publication of application: **03.08.2016**
(21) Application number: 16151186.0
(22) Date of filing: 14.01.2016
(51) Int. Cl.: A61K 8/35, A45D 31/00, A61K 8/37, A61Q 3/02, A61K 8/55, A61K 8/81

(54) **SINGLE STEP PEELING OFF LIGHT NAIL GEL AND MANUFACTURING METHOD THEREOF**

(30) Priority: 27.01.2015 US 201514607058
(71) Applicant: Zhen, Lijuan, City of Industry 91745 (US)
(72) Inventor: Zhen, Lijuan, City of Industry 91745 (US)
(74) Representative: Casalonga

(57) **Abstract**

A nail gel composition includes a nail gel mixture and a color agent mixing therewith for forming a nail gel layer on a nail of a user. The nail gel mixture includes first through sixth chemical elements, wherein the first through sixth chemical elements are polyacrylate-15, di-hema trimethylhexl dicarbamate, phenylbis (2,4,6-trimethylbenzoyl) phosphine oxide, hema, trimethylbenzoyl di-phenylphosphine, and 1-hydroxycyclohexyl phenyl ketone respectively. The nail gel layer is dried under an exposure of one of LED light, UV light, and sunlight that no wet sticky colloid is formed after the nail gel layer is dried with glossy high shine kept on the nail more than 10 days. The nail gel layer is adapted for being removed by directly peeling off the nail gel layer on the nail in one single step.

## Description

### NOTICE OF COPYRIGHT

A portion of the disclosure of this patent document contains material which is subject to copyright protection. The copyright owner has no objection to any reproduction by anyone of the patent disclosure, as it appears in the United States Patent and Trademark Office patent files or records, but otherwise reserves all copyright rights whatsoever.

### BACKGROUND OF THE PRESENT INVENTION

### FIELD OF INVENTION

The present invention relates to a peelable nail gel, and more particularly to a single step peeling off light nail gel and its manufacturing and using methods, wherein the nail gel is a light drying gel that can be directly applied on users' finger or toe nails without using the base coat and color coat nail polishes, be cured and dried out under light structure, including but not limited to UV light, LED light and/or natural sunlight having a predetermined light wave, such as between 385-405 light wave, within approximately 30 seconds to 120 seconds, and be peeled off by a single step after it is applied ten days or more.

### DESCRIPTION OF RELATED ARTS

Conventional nail gel polishs or nail polishs currently found in the market require polish-remover to remove the polish on the nails, such that the polish remover is adapted to dissolve the polish layer on the nails. Sometimes, the users need to apply large amount of polish remover on their nails, or even need to soak their nails in the polish remover for a short period of time in order to allow the polish layers completely dissolved by the polish remover. In other words, the users need to saturate a rag, cotton or tissue paper with the nail polish remover to remove the nail polish, and the removing process of the conventional nail polish remover is inconvenience and troublesome.

However, the nail polish removers in general are based on acetone. Acetone is a flammable solvent and a toxic chemical. Exposure to high levels of acetone may cause death, unconsciousness, and damages of the kidneys and the mouth's skin. Therefore, acetone will penetrate through the skin and nail to the user when the nail polish remover is applied on the nails. It is worth mentioning that acetone may accumulate in the user's body, which is harmful for the user's health. The acetone also has a strong smell, so the users may feel irritations for their nose, throat, lung, and eyes while they are using the nail polish remover. Furthermore, the nails are easily damaged by the acetone.

An improved peelable nail polish is provided in the current market, such that the improved nail gel polish can be peeled off after drying. Accordingly, the users need to inititially apply the peelable base coat nail polish on their nails as a first layer, and apply the color nail polish thereon as a second layer, and then apply one more peelable off top coat as a third layer. Preferably, the color nail polish should be applied on the nail twice for better result. Accordingly, the users need to apply a coat peelable nail polish on the first or third layer in order to achieve the peelable function. In particular, in order to dry each layer on the nail, the nail must be exposed under a LED light or a UV light. In other words, each layer cannot be dried under the sunlight. For example, the nail must be exposed under a LED light for 30 to 60 seconds or a UV light for two mintues in order to dry each layer. In addition, after each layer is dried, some wet sticky colloids will still residued on the layer. Therefore, the user must use alcohol to remove wet sticky colloids on each layer and to polish each layer after the following layer is applied. For such four-layer nail polish structure, the user will spend at least 60 mintues, including nail polish applying time, drying time, and residue removing time, to complete the four layers. It is time-wasting to apply multiple layers of nail polish on the user's nails since the users need to wait until the first layer is dried before the following layer is applied thereon. This multiple-layer nail polish structure can only last between 2 and 10 days. In addition, the users need to purchase not only the color nail polish, but also the base coat and top coat nail polish.

Accordingly, the users need to completely remove the peelable nail polish by soaking the polished nails into the warm water for few minutes in order to peel off the entire nail polish layer. Therefore, the process for applying the improved peelable nail polish on the nails and the process for removing the peelable nail polish from the nails are complicated, and the users need to spend lots of times and money on both processes. At least, the improved peelable nail polish has no need to put nail into warm water before peeling that can just be peeled off anytime you like.

### SUMMARY OF THE PRESENT INVENTION

The invention is advantageous in that it provides a signel step peeling off light nail gel, wherein the nail gel can be easily and quickly applied on users' finger or toe nails directly without using any base coat and top coat nail polish or gel polish, and completely cured and dried out on the nails after exposing under a light structure, including but not limited to UV light, LED light and natual sunlight, within 30 seconds to 120 seconds, with glossy shine surface lasting 10 days or more and without any wet sticky residue on the nail surfaces.

Another advantage of the invention is to provide a signel step peeling off light nail gel, wherein the cured and dried nail gel is able to be directly peeled off from the nails in one signle step after it is applied 10-14 days or more.

Another advantage of the invention is to provide signel step peeling off light nail gel, wherein the nail gel has the longer life-span on the nails comparing to the conventional peelable nail polish, such that the nail gel layer formed by the nail gel of the present invention has the stronger layer structure than that of the conventional nail polish.

Another advantage of the invention is to provide signel step peeling off light nail gel, wherein the nail gel can be peeled off from the nails by hands without any additional solvent remover, so as to reduce the damages for the nails during each nail gel removing process.

Another advantage of the invention is to provide signel step peeling off light nail gel, wherein the users only need to apply no more than two layers, preferrably one layer, of nail gel on the nails to achieve a peelable function and is prone to peeling, so it is time-saving and money-saving for the users without applying and purchasing the peelable base coat and color coat nail polishes.

Another advantage of the invention is to provide signel step peeling off light nail gel, wherein no expensive and complicated structure is required to be employed in the present invention in order to achieve the above mentioned advantages. Therefore, the present invention successfully provides an economic and efficient solution to simply the applying and removing processes for the nail polish.

Additional advantages and features of the invention will become apparent from the description which follows, and may be realized by means of the instrumentalities and combinations particular point out in the appended claims.

According to the present invention, the foregoing and other objects and advantages are attained by signel step peeling off light nail gel composition which comprises a nail gel mixture and a color agent mixing with the nail gel mixture.

The nail gel mixture comprises first through sixth chemical elements, wherein the first through sixth chemical elements are polyacrylate-15, di-hema trimethylhexl dicarbamate, phenylbis (2,4,6-trimethylbenzoyl)phosphine oxide, hema, trimethylbenzoyl di-phenylphosphine, and 1-hydroxycyclohexyl phenyl ketone respectively.

In accordance with another aspect of the invention, the present invention comprises a manufacturing method of a nail gel, comprising the following steps.
(1) Provide first through sixth chemical elements, including a first chemcial element of polyacrylate-15, a second chemcial element of di-hema trimethylhexl dicarbamate, a third chemcial element of phenylbis (2,4,6-trimethylbenzoyl)phosphine oxide, a fourth chemcial element of hema, a fifth chemcial element of trimethylbenzoyl di-phenylphosphine, and a sixth chemcial element of 1-hydroxycyclohexyl phenyl ketone.
(2) Mix and stir the second chemical element with the third chemical element together to form an intital first mixture.
(3) Disslove the first chemical element into the first mixture by heating and stirring the first chemical element in the first mixture to form a second mixture.
(4) Mix and stir the fourth to sixth chemical elements into the second mixture to form a third mixture.
(5) Add and mix a color agent into the third mixture to form a final product of the nail gel.

In accordance with another aspect of the invention, the present invention comprises a method of using a nail gel, comprising the following steps.
(A) Apply a layer of nail gel on a nail.
(B) Dry the layer under an exposure of light having a light wave between 385-405, such as UV light, LED light and sunlight, wherein the nail gel layer is cured and dried between 30-120 seconds and that no wet sticky colloid is formed on the cured and dried nail gel layer.
(C) When removing the layer, directly peel off the layer on the nail in one single step.

Still further objects and advantages will become apparent from a consideration of the ensuing description and drawings.

These and other objectives, features, and advantages of the present invention will become apparent from the following detailed description, the accompanying drawings, and the appended claims.

### BRIEF DESCRIPTION OF THE DRAWINGS

Fig. 1 is a block diagram of a method of manufacturing a nail gel according to a preferred embodiment of the present invention.
Fig. 2 is a block diagram of a method of using a nail gel according to the above preferred embodiment of the present invention.
Fig. 3 illustrates the nail gel layer on the nail and adapted to be peeled of therefrom according to the above preferred embodiment of the present invention.

### DETAILED DESCRIPTION OF THE PREFERRED EMBODIMENT

The following description is disclosed to enable any person skilled in the art to make and use the present invention. Preferred embodiments are provided in the following description only as examples and modifications will be apparent to those skilled in the art. The general principles defined in the following description would be applied to other embodiments, alternatives, modifications, equivalents, and applications without departing from the spirit and scope of the present invention.

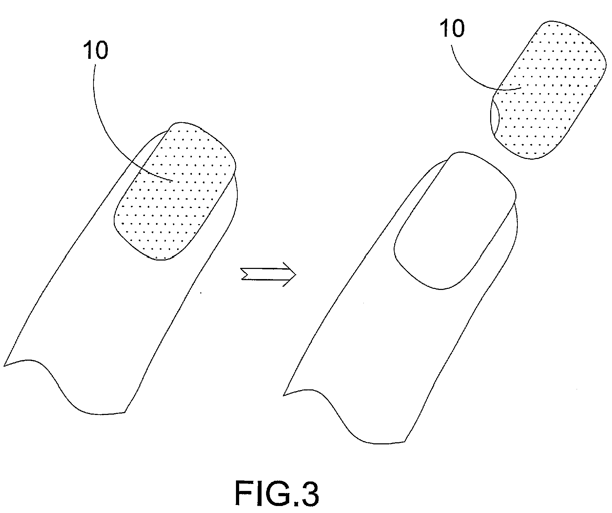

As shown in Fig. 3 of the drawings, a single step peeling off light nail gel of the present invention is adapted for directly applying on a nail of a user and for being quickly cured and dried under an exposure of light. The user is able to remove the layer 10 by directly peeling off the layer 10 on the nail in one single step.

The nail gel according to the preferred embodiment of the present invention is illustrated, wherein the nail gel composition comprises a nail gel mixture and a color agent mixing with the nail gel mixture. The nail gel mixture comprises first through sixth chemical elements, including a first chemical element of polyacrylate-15, a second chemical element of di-hema trimethylhexl dicarbamate, a third chemical element of phenylbis (2,4,6-trimethylbenzoyl) phosphine oxide, a fourth chemical element of hema, a third chemical element of trimethylbenzoyl di-phenylphosphine, and a sixth chemical element of 1-hydroxycyclohexyl phenyl ketone respectively.

Accordingly, an amount of the first chemcial element (polyacrylate-15) has a range of 63-77% by weight. An amount of the second chemcial element (di-hema trimethylhexl dicarbamate) has a range of 11.7-14.3% by weight. An amount of the third chemcial element (phenylbis (2,4,6-trimethylbenzoyl)phosphine oxide) has a range of 5.4-6.6% by weight. An amount of the fourth chemcial element (hema) has a range of 2.7-3.3% by weight. An amount of the fifth chemcial element (trimethylbenzoyl di-phenylphosphine) has a range of 2.7-3.3% by weight. An amount of the sixth chemcial element (1-hydroxycyclohexyl phenyl ketone) has a range of 4.5-5.5% by weight. In particular, a weight ratio of the first through sixth chemcial element is 70:13:6:3:3:5.

Accordingly, the nail gel mixture is a dryable mixture being dried under an exposure of LED light, UV light, or sunlight.

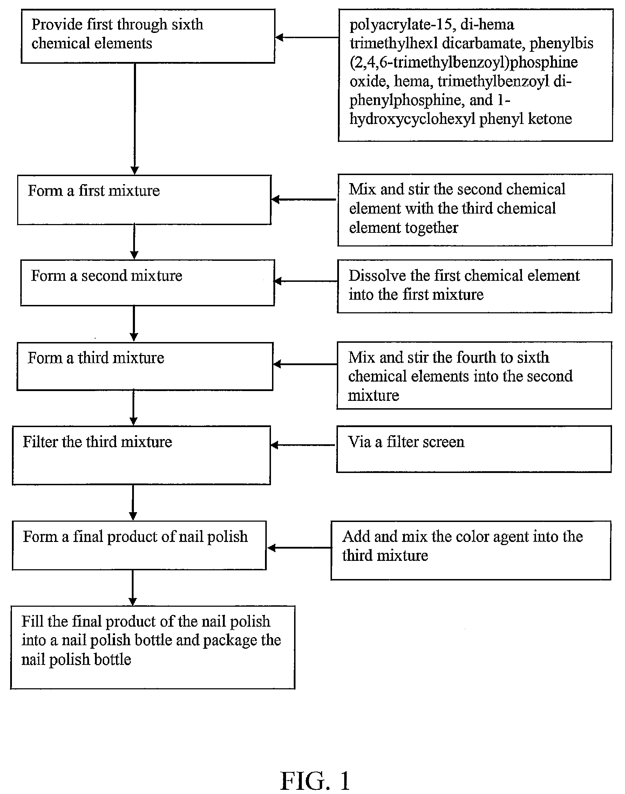

Referring to Fig. 1 of the drawings, the present invention provides a method of manufacturing the nail gel, wherein the manufacturing method comprises the following steps:
(1) Provide the first through sixth chemical elements. Accordingly, the first through sixth chemical elements and their weight ratio are shown above.
(2) Mix and stir the second chemical element with the third chemical element together to form an intital first mixture. Accordingly, the second chemical element and the third chemical element are preferrably stirred for 30 mintues under room temperature (25°C) to form the first mixture.
(3) Dissolve the first chemical element into the first mixture by heating and stirring the first chemical element in the first mixture to form a second mixture. Accordingly, the second mixture is preferrably stirred for 40 mintue under 60°C in order to ensure the first chemical element to be dissloved in the first mixture. In other words, the first mixture is gradually heated up from room temperature to 60°C when the first chemical element is added and disslove into the first mixture.
(4) Mix and stir the fourth to sixth chemical elements into the second mixture to form a third mixture. Accordingly, the fourth to sixth chemical elements are orderly added into the second mixture. In other words, the fourth, fifth, and sixth chemical elements are added into the second mixture in sequence. In addition, the third mixture is preferrably stirred for 30 mintues under room temperature to ensure the fourth to sixth chemical elements are well-mixed into the second mixture. It is worth mentioning that after the first chemical element is dissloved in the first mixture, the second mixture should be cooled down from 60°C back to the room temperature in order to mix with the fourth to sixth chemical elements.
(5) Add and mix the color agent into the third mixture to form a final product of the single step peeling off light nail gel. Accordingly, the color agent contains color pigments to form the final product of the nail gel with a desired color.
(6) Fill the final product of the nail gel into a nail gel bottle and package the nail gel bottle with the nail gel therein, such that the nail gel is ready to use.

According to the preferred embodiment, before the step (5), the manufacturing method further comprises a step of filtering the third mixture to remove residues in the third mixture before the color agent is added thereinto. Accordingly, the third mixture is filtered by screen, such as a 200 mesh gauze screen, to ensure the standardized particle size of the third mixture.

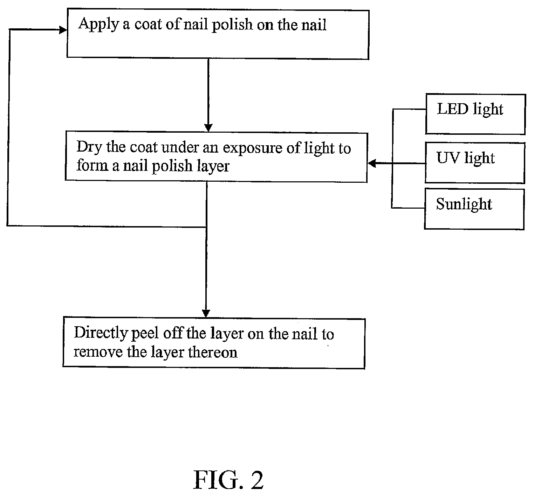

Referring to Fig. 2 of the drawings, the present invention further provides a method of using the nail gel, wherein the using method comprises the following steps.
(A) Apply a coat of nail gel on the nail. The user is able to apply a coat of nail gel from the nail gel bottle via a conventional method. It is worth mentioning that no base coating and/or top polish coating is required before and after the nail gel layer 10 is formed on the nail.
(B) Dry the coat under an exposure of light, having a light wave between 385-405,to form the nail gel layer 10 for 30 to 120 seconds, wherein when the layer 10 is cured and dried, no wet sticky colloid is formed on the layer 10. The layer 10 is completely dried under an exposure of LED light (395-405 light wave) for 30 to 60 seconds, under an exposure of UV light (385 light wave) for 2 mintues, or under an exposure of natural sunlight (containing light wave from 385-405) for 1 to 2 mintues. It is worth mentioning that there is no no wet sticky colloid formed on the cured and dried nail gel layer10, such that the user does not need to use alcohol to remove wet sticky colloids on the layer 10 formed by the conventional nail gel.

In other words, the signel step peeling off light nail gel of the present invention can be easily and quickly applied on users' finger or toe nails directly without using any base coat and top coat nail polish or gel polish, and completely cured and dried out on the nails after exposing under a light, such as UV light, LED light and natual sunlight, having light wave 385-405, within 30 seconds to 120 seconds, with glossy shine surface lasting 10 days or more and without any wet sticky residue on the nail surfaces.

It is worth mentioning that, if necessary, the users may repeat the steps (A) and (B) to apply a second coat of nail gel of the present invention on the nail for thicker coating effect. The second coat is applied on the first layer 10 after the first layer 10 is dried. The second coat is also dried under an exposure of light to integrally form the second layer on the first layer 10 as one single layer structure. The double layered nail gel is opaque and makes the color more intense and even on the nail. It is worth mentioning that no more than two layers 10 of nail gel are required to apply since two layers 10 of nail gel of the present invention is already better than four layers of conventional nail polish.

The cured and dried nail gel layer 10 can be removed easily after 7-14 days from it is applied on the nail simply by one single step (C) of directly peeling off the cured and dried nail gel layer 10 on the nail, preferrably peeling off the whole piece or in serveral pieces manner. In other words, according to the preferred embodiment, the nail gel layer 10 can be kept on the nail for 7 to 14 days as it is originally applied thereon. The nail gel layer 10 on the nail can be directly peel off by hand without any supplemental tool. Accordingly, the entire layer 10 can be slowly peeled off starting from an edge of the nail, as shown in Fig. 3. It is worth mentioning that if the nail gel of the present inveniton is colorless, the peeled off nail gel piece is treansparent too. Accordingly, it is apparent to person who skilled in the art to use a colorless/transpartent nail gel of the present invention as a base coat and then apply the conventional non-peelable colar nail polish thereon, or a top coat applied on top of the conventional nail polish or nail gel polish.

It is appreciated that the single step peeling off light nail gel of the present invention is able to directly peel off from the nails without using nail gel remover or nailfile, so the nail gel of the present invention can prevent the damage of the nails causing by using the nail gel remover or the gliding action of the naifile. And, the user can save lots of money without purchasing nail gel removers, the base coat nail gel, and the color coat nail gel. In other words, the single step peeling off light nail gel of the present invention is a three-in-one nail gel, which has the functions of nail gel, base coat nail gel and color coat nail gel.

It is worth mentioning that the conventional peelable nail polish cannot be dried by exposing under the natural sunlight, and the life-span of the conventional nail gel after applying on the nail can only last for 2 to 10 days. Therefore, the nail gel of the present invention has longer life-span than the conventional peelable nail polish. The conventional peelable nail polish needs to apply at least three to four layers of nail polishes in order to achieve any possible peelable function, and the nail gel of the present invention only needs to apply even one layer of the nail gel on the nail to reach the same effect as the conventional peelable nail polish product so as to save more time and money for the user to apply or remove the nail polish layers on their nails.

One skilled in the art will understand that the embodiment of the present invention as shown in the drawings and described above is exemplary only and not intended to be limiting.

It will thus be seen that the objects of the present invention have been fully and effectively accomplished. The embodiments have been shown and described for the purposes of illustrating the functional and structural principles of the present invention and is subject to change without departure from such principles. Therefore, this invention includes all modifications encompassed within the spirit and scope of the following claims.

## Claims

**1.** A nail gel composition, comprising:
a nail gel mixture comprising at least a first chemical element of polyacrylate-15 and one or more chemical elements selected from a group consisting of a second chemical element of di-hema trimethylhexl dicarbamate, a third chemical element of phenylbis (2,4,6-trimethylbenzoyl) phosphine oxide, a fourth chemical element of hema, a fifth chemical element of trimethylbenzoyl di-phenylphosphine, and a sixth chemical element of 1-hydroxycyclohexyl phenyl ketone.

**2.** The nail gel composition, as recited in claim 1, further comprising a color agent mixing with said nail gel mixture.

**3.** The nail gel composition, as recited in claim 2, wherein an amount of said first chemcial element has a range of 63-77% by weight, an amount of said second chemcial element has a range of 11.7-14.3% by weight, an amount of said third chemcial element has a range of 5.4-6.6% by weight, an amount of said fourth chemcial element has a range of 2.7-3.3% by weight, an amount of said fifth chemcial element has a range of 2.7-3.3% by weight, and an amount of said sixth chemcial element has a range of 4.5-5.5% by weight, wherein said nail gel mixture is a dryable mixture being dried under an exposure of light selected from a group of a UV light, a LED light and sunlight.

**4.** The nail gel composition, as recited in claim 3, wherein a weight ratio of said first through sixth chemcial element is 70:13:6:3:3:5 and said nail gel mixture is a dryable mixture being dried under an exposure of light having a light wave between 385-405.

**5.** The nail gel composition, as recited in claim 4, wherine said light is selected from a group consisting of UV light, LED light and natural sunlight.

**6.** A method of manufacturing a nail gel, comprising the steps of:
(a) providing first through sixth chemical elements, including the first chemical element of polyacrylate-15, the second chemical element of di-hema trimethylhexl dicarbamate, the third chemical element of phenylbis (2,4,6-trimethylbenzoyl) phosphine oxide, the fourth chemical element of hema, the fifth chemical element of trimethylbenzoyl di-phenylphosphine, and the sixth chemical element of 1-hydroxycyclohexyl phenyl ketone, wherein an amount of said first chemcial element has a range of 63-77% by weight, an amount of said second chemcial element has a range of 11.7-14.3% by weight, an amount of said third chemcial element has a range of 5.4-6.6% by weight, an amount of said fourth chemcial element has a range of 2.7-3.3% by weight, an amount of said fifth chemcial element has a range of 2.7-3.3% by weight, and an amount of said sixth chemcial element has a range of 4.5-5.5% by weight;
(b) mixing and stirring said second chemical element with said third chemical element together to form an intital first mixture;
(c) dissloving said first chemical element into said first mixture by heating and stirring said first chemical element in said first mixture to form a second mixture; and
(d) mixing and stirring said fourth to sixth chemical elements into said second mixture to form a third mixture.

**7.** The method, as recited in claim 6, after the step (d), further comprising a step (e) of adding and mixing a color agent into said third mixture to form a final product of said nail gel, and, before the step (d), further comprising a step of filtering said third mixture to remove residues in said third mixture before said color agent is added thereinto, wherein, in the step (b), said second chemical element and said third chemical element are stirred for 30 mintues under room temperature to form said first mixture, wherein, in the step (c), said second mixture is stirred for 40 mintue under 60°C in order to ensure said first chemical element to be dissloved in said first mixture.

**8.** The method, as recited in claim 6, wherein, in the step (b), said second chemical element and said third chemical element are stirred for 30 mintues under room temperature to form said first mixture, wherein, in the step (c), said second mixture is stirred for 40 mintue under 60°C in order to ensure said first chemical element to be dissloved in said first mixture, wherein, in the step (d), said fourth to sixth chemical elements are orderly added into said second mixture and are stirred for 30 mintues under room temperature.

**9.** The method, as recited in claim 6, wherein a weight ratio of said first through sixth chemcial element is 70:13:6:3:3:5.

**10.** A method for using a single step peeling off light nail gel, comprising the steps of:
(a) applying a coat of nail gel on a nail, wherein said nail gel is a mixture of at least a first chemical element of polyacrylate-15 and one or more chemical elements selected from a group consisting of a second chemical element of di-hema trimethylhexl dicarbamate, a third chemical element of phenylbis (2,4,6-trimethylbenzoyl) phosphine oxide, a fourth chemical element of hema, a fifth chemical element of trimethylbenzoyl di-phenylphosphine, and a sixth chemical element of 1-hydroxycyclohexyl phenyl ketone;
(b) drying said coat under an exposure of light, having a light wave between 385-405, to form a nail gel layer for 30-120 seconds, wherein when said nail gel layer is cured and dried, no wet sticky colloid is formed on said layer; and
(c) when removing said layer, directly peeling off said layer from the nail in one single step after 7 days or more from the day of application of the nail gel layer in step (b).

**11.** The method, as recited in claim 10, wherein an amount of said first chemcial element has a range of 63-77% by weight, an amount of said second chemcial element has a range of 11.7-14.3% by weight, an amount of said third chemcial element has a range of 5.4-6.6% by weight, an amount of said fourth chemcial element has a range of 2.7-3.3% by weight, an amount of said fifth chemcial element has a range of 2.7-3.3% by weight, and an amount of said sixth chemcial element has a range of 4.5-5.5% by weight.

**12.** The method, as recited in claim 11, wherein a weight ratio of said first through sixth chemcial element is 70:13:6:3:3:5.

**13.** The method, as recited in claim 10 wherein, in the step (b), said layer is dried under an exposure of light selected from a group selected from a UV light, a LED light and a natural sunlight, wherien when said light is the UV light, said layer is dried under exposure for 2 minutes, wherein when said light is the LED light, said layer is dried under exposure for 30 to 60 seconds, wherein when said light is the sunlight, said layer is dried under exposure for 1 to 2 minutes.

**15.** The method, as recited in claim 12, after the step (b), further comprising a step of repeating the steps (a) and (b), for applying a second coat of the nail gel on said layer of nail gel, wherein, in the step (b), said layer is dried under an exposure of light selected from a group selected from a UV light, a LED light and a natural sunlight, wherien when said light is the UV light, said layer is dried under exposure for 2 minutes, wherein when said light is the LED light, said layer is dried under exposure for 30 to 60 seconds, wherein when said light is the sunlight, said layer is dried under exposure for 1 to 2 minutes,.
